(19) Europäisches Patentamt
European Patent Office
Office européen des brevets

(11) **EP 4 596 102 A1**

(12) **EUROPEAN PATENT APPLICATION**

(43) Date of publication:
06.08.2025 Bulletin 2025/32

(21) Application number: 24155196.9

(22) Date of filing: 01.02.2024

(51) International Patent Classification (IPC):
*B01J 23/42* (2006.01)    *B01J 37/02* (2006.01)
*C07C 21/06* (2006.01)

(52) Cooperative Patent Classification (CPC):
(C-Sets available)
B01J 23/42; B01J 37/0209; C07C 17/08    (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicants:
• Johnson Matthey Public Limited Company
London EC2V 7AD (GB)
• ETH Zurich
8092 Zürich (CH)

(72) Inventors:
• GIULIMONDI, Vera
8064 Zurich (CH)
• JOHNSTON, Peter
Royston, SG8 5HE (GB)
• PÉREZ-RAMÍREZ, Javier
8006 Zurich (CH)
• SMIT, Joost
London, W2 6LG (GB)

(74) Representative: Johnson Matthey Plc
Intellectual Property Department
Belasis Avenue
Billingham TS23 1LB (GB)

(54) **PLATINUM CONTAINING CATALYST, METHOD OF PREPARATION, AND USE**

(57)    The invention provides a hydrochlorination catalyst, a method of manufacturing a hydrochlorination catalyst, and a process for catalytic hydrochlorination of a substrate containing an alkyne unit using the catalyst. The catalyst comprises a complex of Pt (II), wherein the complex is supported on a support .

FIG 1

EP 4 596 102 A1

(52) Cooperative Patent Classification (CPC): (Cont.)

C-Sets
**C07C 17/08, C07C 21/06**

## Description

### Field of the Invention

The present invention relates to platinum containing catalysts, particularly for the conversion of acetylene to vinyl chloride monomer (VCM).

### Background

The hydrochlorination of acetylene to produce VCM as the precursor to polyvinyl chloride (PVC) is currently a large scale industrial process, particularly in coal rich areas such as China and in areas rich in natural gas through natural gas to acetylene routes. Over 20 million tonnes of VCM are produced annually through acetylene hydrochlorination with the vast majority utilising mercuric chloride ($HgCl_2$) catalysts supported on activated carbon.

The mercury catalyst poses significant environmental concerns due to volatile $HgCl_2$ subliming from the catalyst bed, up to 0.6 kg Hg/tonne VCM production. Due to the environmental impact of this process, the recently ratified Minamata convention dictates that all new VCM plants must use mercury free catalysts and in the near future all existing industrial plants must switch to mercury free alternatives. This has revived the commercial interest in using other metals, such as gold and platinum, as a catalyst for this reaction.

Platinum has been reported as a catalyst for acetylene hydrochlorination at least since Hutchings' paper in 1985 (G.J. Hutchings, 'Vapour phase hydrochlorination of acetylene: Correlation of catalytic activity of supported metal chloride catalysts', J. Catal., 96 (1985) 292-295). However, that work and subsequent works focused on metal chloride compounds, which require the use of sensitising chloroplatinic acid (CPA) in preparing the catalyst. This resulted in limited commercial interest due to inherent manufacturing complications.

It is known from CN103623838A (Tianjin University) that a Ru-Pt-Cu catalyst can be used for hydrochlorination synthesis of VCM. The catalyst comprises salts of Ru, Pt and Cu supported on a porous inert carrier. The catalyst is claimed to reduce reaction temperature of acetylene hydrochlorination synthesis of VCM to below 180°C.

CN113649057A (Zhejiang Normal University CJNU) discloses a platinum catalyst for preparing VCM by hydrochlorination of acetylene, wherein the catalyst comprises a carbon nitride carrier and platinum as an active material. The platinum is present on the carrier in an ionic state, being loaded onto the carrier by impregnating the carrier using an aqua regia platinum salt solution, drying the impregnated carrier, and then roasting the dried carrier. The platinum salt comprises chloroplatinic acid, potassium chloroplatinate or platinum tetrachloride.

In US5233108 (Solvay SA), a catalytic system for preparing VCM by hydrochlorination of acetylene is described. The system comprises at least one group VIII metal compound, such as a platinum compound, and an amine hydrochloride having a melting point lower than 25°C. The platinum compound is preferably a platinum chloride.

US2010/063333A1 (also to Solvay SA) also discloses a catalytic system for preparing VCM by hydrochlorination of acetylene, which system is comparable to that of US 5233108 in comprising at least one group VIII metal compound and an amine hydrochloride, but requiring that the group VIII metal compound is provided as a mixture of a platinum (VI) compound with tin (II) chloride, or as mixture of a platinum (II) compound with triphenylphosphine oxide, or as a mixture of a palladium (II) compound with triphenylphosphine. The catalyst systems exemplified in this reference are used in the liquid phase without a support.

Selina K. Kaiser et al., "Nanostructuring unlocks high performance of platinum single-atom catalysts for stable vinyl chloride production", Nature Catalysis, April 2020, vol.3 pages 376-385, discloses platinum single-atom catalysts for stable vinyl chloride production, using CPA as precursor.

There is a need for alternative platinum catalysts effective for the hydrochlorination of alkyne substrates, ideally having improved performance when compared to existing platinum catalysts, and ideally avoiding the use of chlorine-containing precursors (such as CPA) in their production.

### Summary of the Invention

The present inventors have surprisingly found that platinum catalysts effective for hydrochlorination of acetylene to produce VCM can be manufactured using Pt (II) complexes.

A first aspect of the invention relates to a hydrochlorination catalyst comprising a complex of Pt (II), wherein the complex is supported on a support.

As used herein, the term "complex" encompasses both neutral Pt (II) complexes and salts comprising a cation and an anion, in which the Pt (II) is in the cation (a Pt (II) complex cation) and/or the anion (a Pt (II) complex anion). The present inventors have surprisingly found that supported catalysts made using Pt (II) complexes are generally more active as hydrochlorination catalysts when compared to catalysts prepared using Pt (IV) complexes such as CPA.

A second aspect of the invention relates to a method of manufacturing a hydrochlorination catalyst according to the first aspect, the method comprising the step of depositing a complex of Pt (II) onto a support, thus providing a supported catalyst. The Pt (II) complex is preferably deposited by impregnation.

A third aspect the invention relates to a process for catalytic hydrochlorination of a substrate containing an alkyne unit, wherein the reaction is carried out in the presence of a hydrochlorination catalyst according to the first aspect or produced by a method according to the second aspect. The process is particularly suitable when acetylene is used as the substrate, to produce VCM.

Description of the Figures

Figure 1 shows catalytic activity in acetylene hydrochlorination expressed as yield of VCM, of carbon supported 0.2 wt.% Pt catalysts, both inventive and comparative, after 1h on stream, without thermal pre-treatment.

Figure 2 shows time-on-stream performance in acetylene hydrochlorination expressed as yield of VCM, of selected 0.2 wt.% Pt catalysts, both inventive and comparative.

Figure 3 shows time-on-stream performance in acetylene hydrochlorination expressed as yield of VCM, of selected 0.2 wt.% Pt catalysts, both inventive and comparative, at different temperatures.

Figure 4 shows catalytic activity in acetylene hydrochlorination expressed as yield of VCM, of selected 0.2 wt.% (darker bars) and 0.8 wt.% (lighter bars) Pt catalysts according to the invention.

Figure 5 shows catalytic activity in acetylene hydrochlorination expressed as yield of VCM, of carbon supported 0.2 wt.% Pt catalysts, both inventive and comparative, after 1 h on stream, with thermal pre-treatment.

Detailed Description

Sub-headings are included for ease of reference only and are not intended to limit the disclosure.

*Catalyst*

The Pt (II) complex may be a neutral complex. Alternatively, the Pt (II) complex may be provided as a Pt (II) salt comprising a cation and an anion, in which the cation and/or the anion includes a Pt (II) center. It will be appreciated that the Pt (II) center is complexed to one or more ligands. It will be appreciated that the coordination around the Pt (II) center will generally be square planar.

In a preferred embodiment the Pt (II) salt comprises a cation which includes a Pt (II) center, in which one, two, three or four $NH_3$ ligands are coordinated to the Pt (II) center. Catalysts made using such salts have been found to be particularly active as hydrochlorination catalysts.

When the Pt (II) salt comprises a cation which includes a Pt (II) center, any organic or inorganic anion(s) may be used to balance the charge on the cation. Preferred salts include those selected from the group consisting of: sulfate, thiosulfate, carbonate, bicarbonate, citrate, nitrate, hydroxide, acetate, fluoride, chloride, bromide and iodide. Halide salts are preferably avoided on health and safety grounds because these can be sensitizing, and in testing by the inventors halide-free salts also showed an activity benefit. Further preferred salts therefore include those selected from the group consisting of: sulfate, carbonate, bicarbonate, citrate, nitrate and hydroxide.

In a particularly preferred embodiment the Pt (II) salt comprises a cation that is tetraammineplatinum (II) $[(NH_3)_4Pt]^{2+}$. Preferred salts of this type include those selected from the group consisting of: tetraammineplatinum (II) sulfate, tetraammineplatinum (II) thiosulfate, tetraammineplatinum (II) carbonate, tetraammineplatinum (II) bicarbonate, tetraammineplatinum (II) citrate, tetraammineplatinum (II) nitrate, tetraammineplatinum (II) hydroxide, tetraamalphtinum (II) acetate, tetraammineplatinum (II) fluoride, tetraammineplatinum (II) chloride, tetraammineplatinum (II) bromide, and tetraammineplatinum (II) iodide. Preferred salts are the sulfate, thiosulfate, carbonate, bicarbonate, citrate, nitrate, hydroxide and acetate salts of tetraammineplatinum (II). It is further preferred that the Pt (II) salt is selected from the group consisting of: tetraammineplatinum (II) sulfate, tetraammineplatinum (II) bicarbonate, and tetraammineplatinum (II) citrate. These salts have been found to produce catalysts which are even more effective than comparable catalysts prepared using tetraammineplatinum (II) chloride.

It is preferred for the Pt (II) salt to be a salt of which the cation and/or the anion, preferably both, is/are chloride (Cl⁻) free. The present inventors have found that catalysts prepared using Pt (II) salts with chloride free anions and/or cations show better activity in the acetylene to VCM reaction compared to when chloride-containing salts are used in the catalyst synthesis. An additional benefit is that chloride-free platinum salts are less sensitizing (or non-sensitizing) than chloride-containing salts.

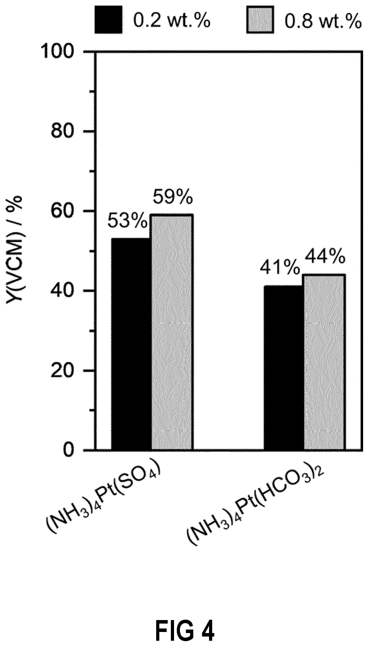

Platinum is a relatively expensive metal and it is therefore preferred that the loading of platinum is kept low. It is preferred that the weight of platinum based on the total weight of catalyst is 0.05 to 1.0 wt.%. The results in Figure 4 of the application demonstrate that, at least in the case of Examples 6 and 11 (prepared using tetraammineplatinum (II) bicarbonate) and Examples 7 and 12 (prepared using tetraammineplatinum (II) sulfate), there is only a relatively small activity increase on increasing the platinum loading from 0.2 wt.% to 0.8 wt.%. A preferred loading of platinum based on the total weight of catalyst is 0.05 to 0.5 wt.% as this provides a good balance between catalyst activity and cost.

The catalyst ideally does not contain metals other than platinum. Therefore, in preferred embodiments the catalyst comprises ≤ 0.1 wt.% of metals other than platinum based on the total weight of catalyst, preferably ≤ 0.05 wt.% of metals other than platinum. In some embodiments the catalyst comprises ≤ 0.02 wt.% of metals other than platinum based on the total weight of catalyst.

The complex is supported on a support. Any known catalyst support may be used to make the catalyst of the invention. Typical metal oxide supports such as alumina, silica, zeolite, silica-alumina, titania or zirconia and composites thereof may be used. It is preferred, however, that the support is a carbon support. The carbon may be derived from natural sources (e.g. peat, wood, coal, graphitic) or may be a synthetic carbon. The carbon is preferably an activated carbon, activated for example by steam, acid, or otherwise chemically activated.

The support may be in the form of a powder, granules or shaped particles. Examples of shaped particles include spheres, tablets, cylinders, multi-lobed cylinders (e.g. trilobes), rings, miniliths, etc. or a massive catalyst unit such as a monolith. Alternatively, the catalyst in the form of a powder may be included in a coating formulation and coated onto a reactor wall or shaped substrate such as a monolith. One preferred form of catalyst support comprises a plurality of shaped units in the form of cylinders, spheres or lobed cylinders each having a diameter of 0.1 to 10 mm, or, more preferably a diameter in the range 1 to 3 mm. In the case of a cross-section shape having a non-uniform diameter, such as a lobed cylinder, the diameter is an average diameter. Cylinders and trilobes are particularly preferred shapes of support.

*Manufacture of the catalyst*

The catalyst may be manufactured by depositing a complex of Pt (II) onto a support, thus providing a supported catalyst. A preferred technique is impregnation, whereby a support is impregnated with an impregnation solution, thus providing an impregnated support. The complex of Pt (II) used in the method of manufacture may be any described under the "catalyst" heading.

Any suitable solvent or solvent mixture may be used to prepare the impregnation solution but a preferred solvent is water, e.g. demineralised water, because this is inexpensive and easy to handle and dispose of.

Any suitable deposition technique may be used. Impregnation techniques are preferred, and incipient wetness impregnation is a preferred technique.

The method may include a subsequent step of drying the impregnated support to remove solvent. Drying techniques will also be well known to those skilled in the art. A typical drying technique involves heating the catalyst at a temperature of > 100°C for a period of 12 hours or more. A stream of gas may be used to help remove the solvent. It will be appreciated that the temperature and duration of drying will differ depending on the scale at which the preparation is carried out.

*Process*

The catalyst of the invention is particularly suitable for hydrochlorination of compounds containing an alkyne unit, especially for the conversion of acetylene to VCM. The conversion of acetylene to VCM is preferably carried out in the gas phase. The conversion of acetylene to VCM is typically carried out at elevated temperature, usually between about 100°C and 250°C. The reaction temperature is a balance between conversion and economics of running the reactor at higher temperatures. Furthermore, at temperatures significantly above 200°C coking can become significant.

A HCl reactant and acetylene are preferably premixed, and also preferably pre-heated to the reaction temperature. Normally HCl is present in excess of the amount required for the stoichiometric reaction. The catalyst may be present in the reactor in the form of a fixed bed of catalyst particles arranged such that the feed gases are passed over or through the catalyst bed. Alternative reactor arrangements may be used, including fluidised beds or other moving bed arrangements. The catalyst may alternatively be provided in the form of a monolith or coated on the wall of a reactor vessel. The catalyst bed may be provided with means to regulate the temperature to avoid overheating due to the exothermic reaction, or to raise the temperature, if required. It may be preferred to treat the catalyst with HCl before use in the process. This treatment is typically carried out by flowing HCl over the catalyst for a period of at least an hour at a temperature of at least 50°C, more especially > 100°C. This pre-treatment may take place in the reactor by operating with a flow of HCl without the acetylene, at a suitable temperature.

Examples

Catalytic performance of carbon supported platinum catalysts for acetylene hydrochlorination

An experimental evaluation of the catalytic performance, in acetylene hydrochlorination, of various carbon supported Pt catalysts, including catalysts according to the invention and comparative catalysts, in extrudate form, was performed. The procedures employed and are discussed in Section 1 - Methods, below. The outcome of the conducted analyses is presented in Section 2 - Results and Discussion, below. Preparation of the catalysts is discussed in Section 3 - Preparation, below.

*Section 1 - Methods*

**1.1. Catalyst pre-treatment**

The catalytic performance in acetylene hydrochlorination of carbon supported Pt catalysts, in extrudate form, prepared using various different platinum (II) or platinum (IV) salts, was evaluated as described in Section 1.2. To investigate the effect of a thermal pre-treatment on catalytic performance, the carbon supported Pt catalysts in extrudate form were thermally pre-treated at 473 K in static air with a heating rate of 5 K min$^{-1}$ and a hold time of 12 h. Their catalytic performance was evaluated as described in Section 1.2 and it is discussed in Section 2.4.

**1.2. Catalytic evaluation**

The gas-phase hydrochlorination of acetylene was performed at ambient pressure in a continuous-flow fixed-bed reactor. The set-up consisted of (i) mass flow controllers to feed acetylene ($C_2H_2$, PanGas, purity >99.6%), hydrogen chloride (HCl, Air Liquide, purity 2.8, anhydrous), and argon (Ar, PanGas, purity >99.999%, internal standard), (ii) an electrically heated oven hosting a quartz micro-reactor (8 mm internal diameter) equipped with a K-type thermocouple whose tip reaches the center of the catalyst bed, (iii) downstream heat tracing to avoid any condensation of the reactants and products, and (iv) a gas chromatograph coupled to mass spectrometer (GC-MS) for on-line analysis.

The carbon supported Pt catalysts in extrudate form were evaluated in the hydrochlorination of acetylene as follows. First, the quartz tubular reactor was loaded with the catalyst (0.25 g), sitting on a plug of quartz wool in the center of the tubular reactor. Prior to testing, the catalyst was heated in a He flow until the desired bed temperature (393 - 473 K), and then stabilized for 15 min under these conditions before flowing the reaction mixture. The reactor was fed with a total volumetric flow of 7.5 - 15 cm$^3$ min$^{-1}$ comprising a gaseous mixture of $C_2H_2$, HCl, and Ar in the following molar ratio 40:44:16 at atmospheric pressure and at the desired catalyst bed temperature. Since vinyl chloride (VCM) was the only product detected in all the tests, the catalytic activity is presented as the yield of VCM, Y(VCM), calculated according to Eq. 1:

$$Y(VCM), \% = \frac{n_{VCM}^{outlet}}{n_{C_2H_2}^{inlet}} \times 100 \qquad \text{Eq. 1}$$

where $n_{VCM}^{outlet}$ and $n_{C_2H_2}^{inlet}$ denote the molar flows of VCM and $C_2H_2$ at the reactor outlet and inlet, respectively. The catalyst initial activity is evaluated by the yield of VCM quantified after 1 h on stream.

The error of the carbon balance, $\varepsilon_C$, determined using Eq. 2, was less than 10% in all experiments, *i.e.*, the carbon mass balance was closed at ≥90%.

$$\varepsilon_C, \% = \frac{n_{C_2H_2}^{inlet} - \left( n_{C_2H_2}^{outlet} + n_{VCM}^{outlet} \right)}{n_{C_2H_2}^{inlet}} \times 100 \qquad \text{Eq. 2}$$

Where $n_{C_2H_2}^{outlet}$ denote the molar flows of $C_2H_2$ at the reactor outlet.

After the tests, the reactor was quenched to room temperature in He flow and the catalyst was retrieved for further characterization.

### 1.3. Catalyst characterization

Scanning transmission electron microscopy (STEM) analysis employing a high-angle annular dark-field (HAADF) detector was conducted on an aberration-corrected HD2700CS (Hitachi) microscope operated at 200 kV.

Thermogravimetric analysis (TGA) was performed using a Linseis STA PT1600 system. TGA of the pre-use and used catalysts was conducted in diluted oxygen (20 vol.% $O_2$/Ar, 100 $cm^3$ $min^{-1}$), heating the samples (amount fixed to 20 mg) from 298 to 1273 K at 10 K $min^{-1}$ to quantify the amount of coke in the used catalysts. The amount of coke formed in the used catalysts, relative to the pre-use one, was quantified by comparing the weight loss in the temperature region of 600 - 700 K.

*Section 2 - Results & Discussion*

### 2.1. Initial catalytic activity

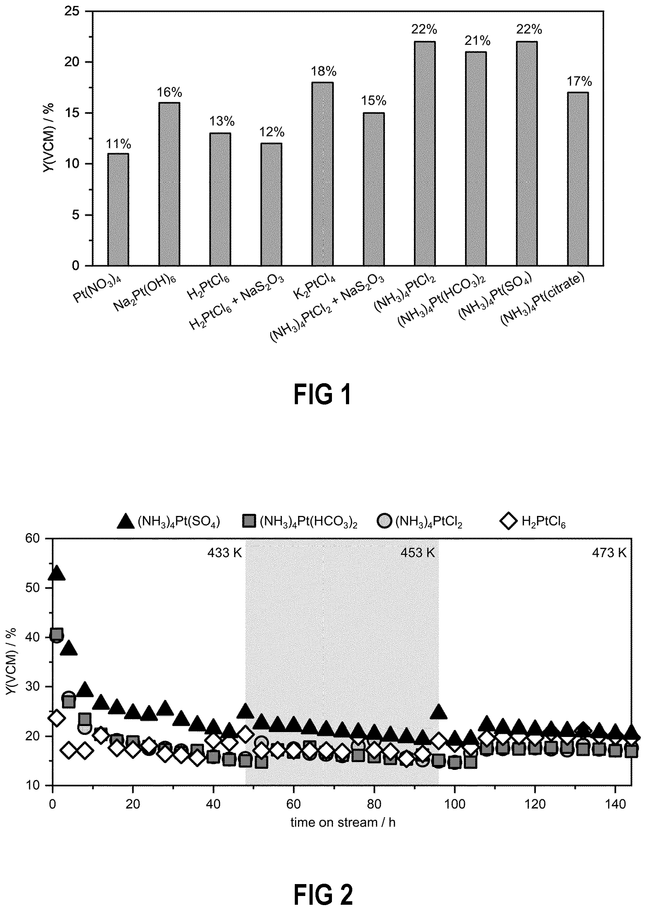

The catalytic activity in acetylene hydrochlorination of the non-thermally pre-treated carbon supported 0.2 wt.% Pt catalysts in extrudate form was first evaluated by quantifying the yield of VCM after 1 h on stream (Figure 1). More specifically, Figure 1 shows initial catalytic activity in acetylene hydrochlorination expressed as yield of VCM, Y(VCM) after 1 h on stream, of non-thermally pre-treated carbon supported 0.2 wt.% Pt catalysts in extrudate form. The x-axis labels represent the Pt precursors employed for synthesizing the catalysts. Prior to testing, the catalysts were heated in a He flow until the desired catalyst bed temperature, and then stabilized for 15 min under these conditions before flowing the reaction mixture. Reaction conditions were as follows: molar ratio of feed composition, $C_2H_2$:HCl:Ar = 40:44:16; total volumetric flow 15 $cm^3$ $min^{-1}$; catalyst mass, 0.25 g; catalyst bed temperature, 473 K; reactor pressure, 1 bar.

The yield of VCM differs among the tested catalysts. The best performing catalysts are those derived from the $(NH_3)_4Pt(SO_4)$, $(NH_3)_4Pt(HCO_3)_2$, and $(NH_3)_4PtCl_2$ precursors, respectively leading to 22, 21, and 22% yield of VCM. This results in a +70% improvement in the catalytic activity with respect to that of the catalyst derived from the $H_2PtCl_6$ precursor, leading to 13% yield of VCM.

The addition of the $Na_2S_2O_3$ salt to the $H_2PtCl_6$ precursor results in virtually unaltered performance: 12% yield of VCM.

Other catalysts presenting improved catalytic activity compared with that of the $H_2PtCl_6$-derived catalyst are those derived from $K_2PtCl_4$, $(NH_3)_4Pt(citrate)$, $Na_2Pt(OH)_6$, and $(NH_3)_4PtCl_2+Na_2S_2O_3$ respectively leading to 18, 17, 16, and 15% yield of VCM. Lastly, the catalyst derived from the $Pt(NO_3)_4$ precursor shows similar performance to that of the $H_2PtCl_6$-derived counterpart, *i.e.*, 11% yield of VCM.

### 2.2. Time on stream performance

The performance of the catalysts yielding the highest initial activity, namely those derived from $(NH_3)_4Pt(SO_4)$, $(NH_3)_4Pt(HCO_3)_2$, and $(NH_3)_4PtCl_2$ precursors, and that of the reference catalyst, derived from the $H_2PtCl_6$ precursor, was evaluated over extended time on stream and under different, increasing catalyst bed temperature (Figure 2). More specifically, Figure 2 shows time-on-stream performance in acetylene hydrochlorination expressed as yield of VCM, Y(VCM), of selected non-thermally pre-treated carbon supported 0.2 wt.% Pt catalysts in extrudate form. The labels represent the Pt precursors employed for synthesizing the catalysts. Prior to testing, the catalysts were heated in a He flow until the desired bed temperature, and then stabilized for 15 min under these conditions before flowing the reaction mixture. To evaluate the performance under different temperatures, the catalyst bed temperature was varied from 433 to 473 K, with a 20 K increment.

The catalyst was maintained at every bed temperature for 48 h. Reaction conditions were as follows: molar ratio of feed composition, $C_2H_2$:HCl:Ar = 40:44:16; total volumetric flow 7.5 $cm^3$ $min^{-1}$; catalyst mass, 0.25 g; reactor pressure, 1 bar. Furthermore, the total volumetric flow was halved with respect to the initial activity tests described above (Figure 1), to reduce the space velocity, increase the yield of VCM, and ultimately enhance the differences in performance among catalysts.

The $(NH_3)_4Pt(SO_4)$-derived catalyst exhibited the highest initial activity, with 53% yield of VCM after 1 h on stream; followed by the $(NH_3)_4Pt(HCO_3)_2$- and $(NH_3)_4PtCl_2$-derived catalysts, leading to 41 and 40% yield of VCM after 1 h on stream, respectively. The reference $H_2PtCl_6$-derived catalyst showed the lowest initial activity, with 24% yield of VCM after 1 h on stream.

Over time on stream, all catalysts exhibit a loss in activity. After 12 h on stream the VCM yield of the $(NH_3)_4Pt(HCO_3)_2$- and $(NH_3)_4PtCl_2$-derived catalysts converges to that of the $H_2PtCl_6$-derived catalyst, at *ca.* 18%.

Although experiencing a loss in activity within the first 12 h on stream, the performance of the $(NH_3)_4Pt(HCO_3)_2$- and $(NH_3)_4PtCl_2$-derived catalysts remains stable over extended time on stream, even upon increasing the bed temperature. The $(NH_3)_4Pt(SO_4)$-derived catalyst also undergoes a loss in activity. However, the $(NH_3)_4Pt(SO_4)$-derived

catalyst exhibits 24% yield of VCM after 12 h on stream as opposed to the 18% yield of VCM of the $(NH_3)_4Pt(HCO_3)_2$- and $(NH_3)_4PtCl_2$-derived counterparts. After 100 h on stream, upon reaching a catalyst bed temperature of 473 K, its catalytic performance matches that of the $H_2PtCl_6$-derived reference on stream, at *ca.* 20% yield of VCM.

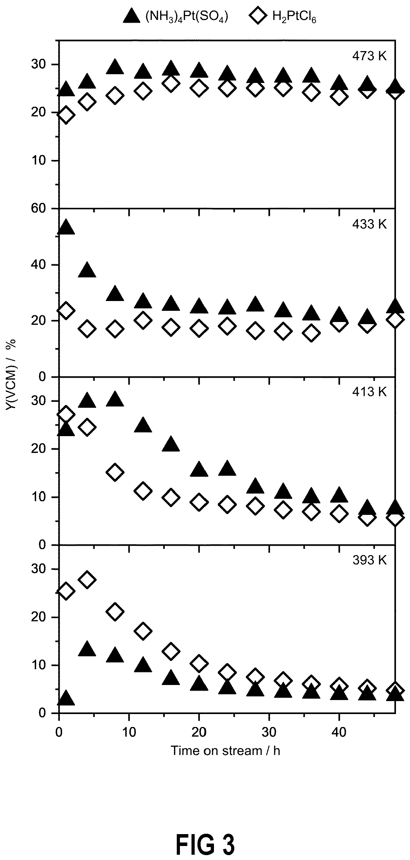

The performance of the $(NH_3)_4Pt(SO_4)$-derived catalyst was evaluated at different temperatures, ranging from 393 to 413 K, and compared to that of the reference $H_2PtCl_6$-derived counterpart (Figure 3). More specifically, Figure 3 shows time-on-stream performance in acetylene hydrochlorination expressed as yield of VCM, Y(VCM), of selected non-thermally pre-treated carbon supported 0.2 wt.% Pt catalysts in extrudate form, evaluated at different temperatures. The labels represent the Pt precursors employed for synthesizing the catalysts. Prior to testing, the catalysts were heated in a He flow until the desired bed temperature, and then stabilized for 15 min under these conditions before flowing the reaction mixture. Reaction conditions were as follows: molar ratio of feed composition, $C_2H_2$:HCl:Ar = 40:44:16; total volumetric flow 7.5 $cm^3$ $min^{-1}$; catalyst mass, 0.25 g; catalyst bed temperature: 413 to 473 K; reactor pressure, 1 bar.

The $(NH_3)_4Pt(SO_4)$-derived catalyst exhibits higher initial activity than the reference $H_2PtCl_6$-derived counterpart, in the temperature range of 413 to 473 K. The most pronounced difference in the initial catalytic activity of the two catalysts is observed at 433 K, where the $(NH_3)_4Pt(SO_4)$- and $H_2PtCl_6$-derived catalysts respectively exhibit 53% and 24% yield of VCM after 1 h on stream. At 393 K, the $(NH_3)_4Pt(SO_4)$-derived catalyst exhibits lower activity than the reference $H_2PtCl_6$-derived counterpart, respectively leading to 25 and 3% yield of VCM after 1 h on stream. Regardless of the bed temperature, the catalytic performance of the $(NH_3)_4Pt(SO_4)$-derived catalyst eventually matches that of the reference $H_2PtCl_6$-derived counterpart over time on stream, after ca. 48 h.

The amount of coke formed over the $(NH_3)_4Pt(SO_4)$-derived catalyst under reaction conditions was evaluated after 48 h on stream upon testing at two different temperatures, 473 K and 433 K. Coke formation was quantified by thermogravimetric analysis of the pre-use and used catalysts (Figure 4), showing a small amount of carbonaceous deposits: 2.0 wt.% upon use at 473 K and 2.8 wt.% upon use at 433 K. More specifically, Figure 4 shows initial catalytic activity in acetylene hydrochlorination expressed as yield of VCM, Y(VCM) after 1 h on stream, of selected non-thermally pre-treated carbon supported 0.2 wt.% (black bars) and 0.8 wt.% (gray bars) Pt catalysts in extrudate form. The catalyst mass was varied to maintain the reactant flow rate per number of metal sites constant. Prior to testing, the catalysts were heated in a He flow until the desired catalyst bed temperature, and then stabilized for 15 min under these conditions before flowing the reaction mixture. Reaction conditions were as follows: molar ratio of feed composition, $C_2H_2$:HCl:Ar = 40:44:16; total volumetric flow 7.5 $cm^3$ $min^{-1}$; catalyst mass, 0.25 g for the catalyst with 0.2 wt.% metal loading and 0.06 g for the catalyst featuring 0.8 wt.% metal loading; catalyst bed temperature, 433 K; reactor pressure, 1 bar.

Table 1, below, shows the amount of coke formed over the carbon supported 0.2 wt.% Pt catalyst in extrudate form derived from the $(NH_3)_4Pt(SO_4)$ precursor after use in acetylene hydrochlorination for 48 h on stream at 473 K and 433 K, as determined by thermogravimetric analysis (TGA). The catalyst was analyzed in its pre-use form and after use, in 20 vol.% $O_2$/Ar. The amount of coke formed in the used catalysts, relative to the pre-used ones, was quantified by comparing the weight loss in the temperature region of 600 - 700 K. The used catalysts were tested under the following reaction conditions: molar ratio offeed composition, $C_2H_2$:HCl:Ar = 40:44:16; total volumetric flow 7.5 $cm^3$ $min^{-1}$; catalyst mass, 0.25 g; catalyst bed temperature, 473 or 433 K; reactor pressure, 1 bar.

**Table 1:** Amount of coke formed over the carbon supported 0.2 wt.% Pt catalyst

| Catalyst bed temperature / K | Relative amount of coke formed after 48 h on stream / wt.% |
|---|---|
| 433 | 2.0 |
| 473 | 2.8 |

## 2.3. Metal loading effect on catalytic activity

The effect of metal loading on catalytic activity was investigated for two catalysts: the $(NH_3)_4Pt(SO_4)$- and the $(NH_3)_4Pt(HCO_3)_2$-derived ones, presenting a nominal Pt content of 0.2 and 0.8 wt.%. The catalysts with different metal loading were tested by adjusting the catalyst mass to maintain the reactant flow rate per number of metal sites constant (Figure 4). Namely, the catalyst mass was reduced by three-quarters when testing the catalysts with 0.8 wt.% metal loading. The catalytic tests show similar initial activity between the samples with a nominal Pt content of 0.2 and 0.8 wt.%.

## 2.4. Thermal pre-treatment effect on catalytic activity

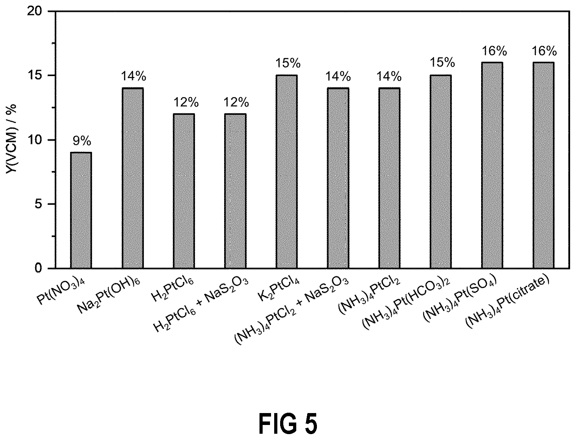

The effect of a mild thermal pre-treatment of the non-thermally pre-treated carbon supported 0.2 wt.% Pt catalysts in extrudate form on their catalytic activity in acetylene hydrochlorination was evaluated by quantifying the yield of VCM after 1 h on stream (Figure 5). More specifically, Figure 5 shows initial catalytic activity in acetylene hydrochlorination expressed as yield of VCM, Y(VCM) after 1 h on stream, of the carbon supported 0.2 wt.% Pt catalysts in extrudate form thermally pre-treated at 473 K in static air with a heating rate of 5 K min$^{-1}$ and a hold time of 12 h. The x-axis labels represent the Pt precursors employed for synthesizing the catalysts. Prior to testing, the catalysts were heated in a He flow until the desired catalyst bed temperature, and then stabilized for 15 min under these conditions before flowing the reaction mixture. Reaction conditions were as follows: molar ratio of feed composition, $C_2H_2$:HCl:Ar = 40:44:16; total volumetric flow 15 cm$^3$ min$^{-1}$; catalyst mass, 0.25 g; catalyst bed temperature, 473 K; reactor pressure, 1 bar.

The yield of VCM differs among the tested catalysts, although the differences are less prominent than those observed for the non-thermally pre-treated catalysts, shown in Figure 1. The best performing catalysts are those derived from the $(NH_3)_4Pt(SO_4)$ and $(NH_3)_4Pt(HCO_3)_2$ precursors, respectively exhibiting 16 and 15% yield of VCM. This results in a +30% improvement in the catalytic activity with respect to that of the reference catalyst derived from the $H_2PtCl_6$ precursor, leading to 12% yield of VCM.

The addition of the $Na_2S_2O_3$ salt to the $H_2PtCl_6$ precursor results in virtually unaltered performance: 12% yield of VCM. Other catalysts presenting improved catalytic activity compared with that of the $H_2PtCl_6$-derived catalyst are those derived from $Na_2Pt(OH)_6$, $(NH_3)_4PtCl_2$, $(NH_3)_4PtCl_2+Na_2S_2O_3$, $K_2PtCl_4$, $(NH_3)_4Pt(citrate)$, and, respectively leading to 14, 14, 14, 15, and 16% yield of VCM. Lastly, the catalyst derived from the $Pt(NO_3)_4$ precursor shows similar performance to that of the $H_2PtCl_6$-derived counterpart, *i.e.*, 9% yield of VCM.

*Section 3 - Preparation*

The extrudate supported catalysts referenced in Section 1 and Section 2, above, were prepared as described below.

For ease of reference, the catalysts, which are characterised in Section 1 and Section 2, above, with reference to metal loading (in wt.%) and precursor, are, in this section, numbered with sample numbers as set out in Table 2, below, with reference to which sample numbers their preparation is subsequently described.

**Table 2:** Extrudate supported catalysts

| Sample | Pt Loading (wt.%) | Precursor |
|---|---|---|
| 1 | 0.20% | $K_2PtCl_4$ |
| 2* | 0.20% | $H_2PtCl_6$ |
| 3 | 0.20% | $[(NH_3)_4Pt]Cl_2$ |
| 4* | 0.20% | $Na_2Pt(OH)_6$ |
| 5* | 0.20% | $Pt(NO_3)_4$ |
| 6 | 0.20% | $[(NH_3)_4Pt](HCO_3)_2$ |
| 7 | 0.20% | $[(NH_3)_4Pt](SO_4)$ |
| 8 | 0.20% | $[(NH_3)_4Pt](citrate)$ |
| 9* | 0.20% | $H_2PtCl_6+Na_2S_2O_3$ |
| 10 | 0.20% | $[(NH_3)_4Pt]Cl_2+Na_2S_2O_3$ |
| 11 | 0.80% | $[(NH_3)_4Pt](HCO_3)_2$ |
| 12 | 0.80% | $[(NH_3)_4Pt](SO_4)$ |
| * not according to the invention | | |

The support used in each example was a carbon extrudate (Norit ROX) which was dried at 105°C in air for 24 hours prior to use.

All metal impregnations were performed by incipient wetness technique in which the Pt containing solution impregnation was done at full pore volume.

**Series 1: Samples 1, 2, 3, 4, 5, 8 - 0.20% Pt/C Catalyst Samples**

The appropriate quantity of Pt precursor complex (as either salt or solution) containing 0.100 g Pt was weighed out and dissolved / diluted in 53 ml demineralised water at ambient temperature. Thereafter, this solution was added dropwise to 50.0 g pre-dried carbon extrudate with agitation / mixing. After all the solution had been added, the material was allowed to stand for ca. 30 min, after which there was no excess or surface solution. Thereafter the material was transferred to a drying dish and dried at 110C in air for ca. 16 hours.

**Sample 6 - 0.20% Pt/C Catalyst Sample**

Sample 6 was prepared as for the samples in series 1 except that the appropriate quantity of $[(NH_3)_4Pt](HCO_3)_2$ precursor complex containing 0.100g Pt was weighed out and dissolved in 53 ml hot demineralised water.

**Sample 7 - 0.20%Pt/C Catalyst Sample**

Sample 7 was prepared as for the samples in series 1 except that the appropriate quantity of $[(NH_3)_4Pt](HCO_3)_2$ precursor complex containing 0.100 g Pt was weighed out and dissolved in 53 ml hot demineralised water. To this solution sufficient $H_2SO_4$ was added dropwise until the solution pH reached 3.6 to form the desired $[(NH_3)_4Pt](SO_4)$ complex.

**Sample 9, 10 - 0.20%Pt/C Catalyst Samples**

Samples 9 and 10 were prepared as for the samples in series 1 except that the Pt precursor complex was diluted in 26 ml demineralised water and sodium thiosulfate (0.081 g) was separately diluted in 27 ml demineralised water and the solutions were mixed together to give an impregnation solution in which the $Pt:Na_2S_2O_3$ molar ratio was 1:1

**Sample 11 - 0.80%Pt/C Catalyst Sample**

Sample 11 was prepared as for Sample 6, except that precursor solution containing 0.400 g Pt was used.

**Sample 12 - 0.80%Pt/C Catalyst Sample**

Sample 12 was prepared as for Sample 7, except that precursor solution containing 0.400 g Pt was used.

**Claims**

1. A hydrochlorination catalyst comprising a complex of Pt (II), wherein the complex is supported on a support.

2. The catalyst according to claim 1, wherein the Pt (II) complex is a Pt (II) salt comprising a cation and an anion, in which the cation and/or the anion includes a Pt (II) center.

3. The catalyst according to claim 2, wherein the Pt (II) salt comprises a cation which includes a Pt (II) center, in which one, two, three or four $NH_3$ ligands are coordinated to the Pt (II) center.

4. The catalyst according to claim 2, wherein the Pt (II) salt comprises a cation that is tetraammineplatinum (II) $[(NH_3)_4Pt]^{2+}$.

5. The catalyst according to any one of claims 2 to 4, wherein the cation and/or the anion of the Pt (II) salt is chloride-free.

6. The catalyst according to any one of claims 2 to 4, wherein the cation and the anion of the Pt (II) salt are chloride-free.

7. The catalyst according to claim 2, wherein the Pt (II) salt is selected from the group consisting of: tetraammineplatinum (II) sulfate, tetraammineplatinum (II) thiosulfate, tetraammineplatinum (II) carbonate, tetraammineplatinum (II) bicarbonate, tetraammineplatinum (II) citrate, tetraammineplatinum (II) nitrate, tetraammineplatinum (II) hydroxide, tetraammineplatinum (II) acetate, tetraammineplatinum (II) fluoride, tetraammineplatinum (II) chloride, tetraammineplatinum (II) bromide, and tetraammineplatinum (II) iodide.

8. The catalyst according to claim 2, wherein the Pt (II) salt is selected from the group consisting of: tetraammineplatinum (II) sulfate, tetraammineplatinum (II) bicarbonate, and tetraammineplatinum (II) citrate.

9. The catalyst according to any of claims 1 to 8, wherein the support is a carbon support.

10. The catalyst according to any one of claims 1 to 9, wherein the weight of platinum based on the total weight of catalyst is 0.05 to 1.0 wt.%.

11. The catalyst according to any one of claims 1 to 9, wherein the weight of platinum based on the total weight of catalyst is 0.05 to 0.5 wt.%.

12. The catalyst according to any one of claims 1 to 11, wherein the catalyst comprises ≤ 0.1 wt.% of metals other than platinum, based on the total weight of catalyst.

13. A method of manufacturing a hydrochlorination catalyst according to any one of claims 1 to 12, the method comprising depositing a complex of Pt (II) onto a support, thus providing a supported catalyst.

14. A process for catalytic hydrochlorination of a substrate containing an alkyne unit, wherein the reaction is carried out in the presence of a hydrochlorination catalyst according to any one of claims 1 to 12 or produced by a method according to claim 13.

15. The process according to claim 14, wherein the substrate is acetylene.

**FIG 1**

**FIG 2**

FIG 3

FIG 4

FIG 5

## EUROPEAN SEARCH REPORT

**Application Number**

EP 24 15 5196

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 116 422 376 A (UNIV SHIHEZI) 14 July 2023 (2023-07-14) | 1,2,9-15 | INV. B01J23/42 |
| Y | * abstract * * claims 1-4; examples 1-5 * | 1-15 | B01J37/02 C07C21/06 |
| X | CN 103 113 412 A (KUNMING INST PRECIOUS METALS) 22 May 2013 (2013-05-22) * abstract * * claims 1,2; example 3 * | 1-8, 10-13 | |
| X | US 2010/285392 A1 (ELABD YOSSEF A [US] ET AL) 11 November 2010 (2010-11-11) * paragraph [0175] * | 1-9,13 | |
| Y | KR 2018 0003351 A (LOTTE FINE CHEMICAL CO LTD [KR] ET AL.) 9 January 2018 (2018-01-09) * abstract * * paragraph [0053] * | 1-15 | |

**TECHNICAL FIELDS SEARCHED (IPC)**

B01J
C07C

The present search report has been drawn up for all claims

| Place of search | Date of completion of the search | Examiner |
|---|---|---|
| Munich | 9 July 2024 | Fischbach, Malaika |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 24 15 5196

09-07-2024

| Patent document cited in search report | Publication date | Patent family member(s) | | Publication date |
|---|---|---|---|---|
| CN 116422376 A | 14-07-2023 | NONE | | |
| CN 103113412 A | 22-05-2013 | NONE | | |
| US 2010285392 A1 | 11-11-2010 | US | 2010285392 A1 | 11-11-2010 |
| | | US | 2013309595 A1 | 21-11-2013 |
| | | WO | 2009045879 A2 | 09-04-2009 |
| KR 20180003351 A | 09-01-2018 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103623838 A
- CN 113649057 A
- US 5233108 A
- US 2010063333 A1

**Non-patent literature cited in the description**

- **G.J. HUTCHINGS**. Vapour phase hydrochlorination of acetylene: Correlation of catalytic activity of supported metal chloride catalysts. *J. Catal.*, 1985, vol. 96, 292-295
- **SELINA K. KAISER et al.** Nanostructuring unlocks high performance of platinum single-atom catalysts for stable vinyl chloride production. *Nature Catalysis*, April 2020, vol. 3, 376-385